Europäisches Patentamt

European Patent Office

Office européen des brevets

(11) Veröffentlichungsnummer: **0 019 793**
**B1**

(12) **EUROPÄISCHE PATENTSCHRIFT**

(45) Veröffentlichungstag der Patentschrift: **21.01.87**

(21) Anmeldenummer: **80102640.2**

(22) Anmeldetag: **13.05.80**

(51) Int. Cl.⁴: **A 61 B 8/06,** G 01 F 1/66, G 01 P 5/00

(54) **Verfahren zur Bestimmung der Geschwindigkeit von bewegter Materie, insbesondere im Körper, und Vorrichtung zu dieser Bestimmung und zur Darstellung von Teilen des Körpers.**

(30) Priorität: **14.05.79 US 38661**

(43) Veröffentlichungstag der Anmeldung:
**10.12.80 Patentblatt 80/25**

(45) Bekanntmachung des Hinweises auf die Patenterteilung:
**21.01.87 Patentblatt 87/04**

(84) Benannte Vertragsstaaten:
**AT BE CH DE FR GB IT LI LU NL SE**

(56) Entgegenhaltungen:
EP-A-0 000 067
AT-B- 285 031
DE-A-2 719 866
DE-A-2 911 613
US-A-3 939 707
US-A-3 958 559
US-A-4 103 679

(73) Patentinhaber: **NEW YORK INSTITUTE OF TECHNOLOGY**
**Wheatley Road**
**Old Westbury, New York 11568 (US)**

(72) Erfinder: **Glenn, Dr. William, E**
**650 Royal Plaza**
**Fort Lauderdale, Florida 33301 (US)**

(74) Vertreter: **Schütz, Peter, Dipl.-Ing. et al**
**Patentanwälte Dr. Dieter v. Bezold Dipl.-Ing.**
**Peter Schütz Dipl.-Ing. Wolfgang Heusler Maria-Theresia-Strasse 22 Postfach 86 02 60**
**D-8000 München 86 (DE)**

Courier Press, Leamington Spa, England.

**Beschreibung**

Die Erfindung betrifft ein Betriebsverfahren für eine Vorrichtung zur Bestimmung der Geschwindigkeit von bewegter Materie im Körper, mit einem einen Ultraschallwendler aufweisenden Meßkopf, der ein fokussiertes Ultraschallstrahlenbündel mit einer gegebenen Apertur auf die Hautoberfläche des Körpers ausstrahlt und das reflektierte Ultraschallstrahlenbündel über die gleiche Apertur aus dem Körper wieder empfängt und mit einer Signalverarbeitungsschaltung, welche Schwebungsfrequenzsignale, die aus von der bewegten Materie reflektierten Dopplereffektkomponenten resultieren, ermittelt und zu einer Geschwindigkeitsanzeige verarbeitet.

In der klinischen Diagnose haben in den vergangenen Jahren Ultraschalltechniken zunehmend an Bedeutung gewonnen, z.B. auf den Gebieten der Gynäkologie, Neurologie und Kardiologie, und in zunehmendem Maße bei der Darstellung verschiedener Körperteile, wie etwa der weiblichen Brust zur Entdeckung von Tumoren. Da es sich bei Ultraschall um mechanische Wellen handelt, unterscheidet er sich von anderen Strahlungsformen bezüglich seiner Wechselwirkung mit lebenden Systemen, und die mit Hilfe von Ultraschall gewonnene Information ist daher in ihrer Natur verschieden von der, die man mit anderen Methoden erhält. Ultraschallverfahren ergänzen andere diagnostische Methoden, wie Röntgenuntersuchungen, bei denen das Risiko von Gewebeschäden nach dem Stand der Erkenntnisse wesentlich größer ist.

Die meisten diagnostischen Verfahren, bei denen Ultraschallenergie benutzt wird, basieren auf der Impulsechomethode, bei der mit einem piezoelektrischen Schallwandler, beispielsweise aus Blei-Zirkonat-Titanat-Keramik, periodisch kurze Ultraschallimpulse erzeugt werden, die jeweils zu einem engen Strahlenbündel fokussiert und in den Körper des Patienten ausgesandt werden. Dort trifft der Impuls auf Grenzflächen zwischen einer Vielzahl verschiedener Strukturen des Körpers. Wenn an einer Grenzfläche eine charakteristische Fehlanpassung der Impedanz vorliegt, wird ein Teil der Ultraschallenergie an dieser Grenze zurück zum Schallwandler reflektiert. Nach Aussenden eines Impulses wird der Wandler auf Empfang geschaltet, so daß er die empfangene reflektierte Energie, d.h. die Echos aus dem Körper, in elektrische Signale zurückverwandelt. Der Zeitpunkt des Eintreffens dieser Echos hängt von der Tiefe der Grenzflächen, auf die der Strahl getroffen ist, und von der Ausbreitungsgeschwindigkeit des Ultraschalls ab. Außerdem ist die Amplitude des Echos ein Anzeichen für die Reflektionseigenschaften der Grenzfläche und demzufolge für die Art der betreffenden Strukturen.

Die Echoinformation kann in verschiedener Weise dargestellt werden. Bei einer gebräuchlichen Methode werden die elektrischen Echosignale verstärkt und an die Vertikalablenkplatten eines Kathodenstrahl - Sichtgerätes angelegt, dessen Horizontalablenkplatten mit dem Ausgang eines Zeitbasisgenerators verbunden sind. Bei dieser sogenannten A-Abtastung zeigt die Horizontalachse die Tiefe der Grenzflächen an, und die Höhe der vertikalen Ablenkungen entspricht der Stärke des Echos. Bei einer anderen Darstellungsform, der sogenannten B-Abtastung, wird die Helligkeit des Bildschirms an jedem Punkt der Abtastung moduliert, wobei die Darstellung einem gewöhnlichen Fernsehbild ähnlich ist. Diese Art der Darstellung ist insbesondere dann sinnvoll, wenn der Ultraschallstrahl eine Abtastbewegung quer zum Körper macht und jeweils eine Tiefeninformation in einer einzelnen Zeile des Sichtgeräts abgebildet wird. Auf einanderfolgende in Querrichtung nebeneinanderliegende Positionen des Ultraschallstrahls ergeben dabei aufeinanderfolgende Zeilen des Sichtgeräts. Die zweidimensionale B-Abtastung liefert ein Querschnittsbild in der Ebene der Abtastung und das resultierende Bildschirmbild kann direkt betrachtet oder photographische bzw. auf Magnetband gespeichert werden.

Neben der Anwendung zu der beschriebenen Darstellung innerer Körperstrukturen wurde Ultraschall unter Auswertung des Doppler - Effektes auch schon zur Messung der Geschwindigkeit bewegter Materie in einem Körper angewendet, wie aus der US—A—4 103 679 bekannt ist. Typischerweise wird hierbei ein Ultraschallstrahlenbündel derart schräg zur Hautoberfläche ausgesandt, daß es eine Komponente in der Richtung (oder auch entgegen der Richtung) des im Körper fließenden Blutes hat. Die aus dem Körper reflektierten Ultraschallechos haben in einem solchen Fall eine als Funktion der Blutgeschwindigkeit durch Doppler-Effekt verschobene Frequenzkomponente.

Die Aufgabe der Erfindung besteht in der Angabe von Maßnahmen, welche bei unkritischerer Handhabung der Meßvorrichtung zu genaueren Meßergebnissen führen.

Diese Aufgabe wird ausgehend von einen Verfahren der eingangs genannten Art, wie sie im Oberbegriff des Anspruchs 1 beschrieben ist, erfindungsgemäß dadurch gelöst, daß der Meßkopf das daß der Zentralstrahl des Strahlenbündels im wesentlichen senkrecht zu der Haut des Körpers gerichtet wird und die reflektierten Ultraschallstrahlen mit dem gleichen, zum Aussenden benutzen Ultraschallwandler empfangen werden und daß die Schwebungsfrequenzsignale, die sich aus den Überlagerung der oberhalb und oder unterhalb der Aussendefrequenz des Ultraschallstrahlenbündels liegenden Dopplereffektkomponenten ergeben zu der Geschwindigkeitsanzeige verarbeitet werden.

Bei dieser senkrechten Einstrahlung lassen sich Fehler bei der Auswertung viel leichter vermeiden, die bei Änderungen eines schrägen Einstrahlwinkels das Ergebnis verfälschen könnten.

Außeredem können bei der senkrechten Einstrahlung des Ultraschallstrahlenbündels der Doppler - Effekt von mit und gegen die Strömungsrichtung geneigten Randstrahlen, also Doppler - komponenten oberhalb und unterhalb der Aussendefrequenz des Ultraschallstrahles, ausgewertet werden, wobie man nicht nur eine größere Genauigkeit, sondern auch eine größere Empfindlichkeit als in Fall einer Auswertung nur einer Dopplerkomponente gegenüber de Ultraschallsendefrequenz enthält.

Das erfindungsgemäße Meßverfahren eignet sich aber auch besonders gut zur Kombination mit einem etwa aus der EP—A1—0 000 067 bekannten Ultraschalldiagnoseverfahren, bei welchem eine Ultraschallabbildung des Körperinneren erfolgt. Ein Kombinationsgerät, welches sowohl eine derartige Darstellung wie auch die Strömungsgeschwindigkeit von Blut in Adern gestattet, ist zwar grundsätzlich schon aus de DE—A1 27 19 866 bekannt, jedoch werden dort für diese beiden Funktionen separate Ultraschallwandler benutzt. Wenn man aber diese beiden Wandler zusammen betreibt, kann es zu Interferenzerscheinungen kommen. Um dies zu vermeiden, kann man für eine simultane Darstellung der Information ohne störende Wechselwirkung das B-Abtastbild speichern und wiederholt darstellen, während der andere Schallwandler in Betrieb ist, um die Dopplereffekt - Geschwindigkeitsinformation zu erhalten, und so eine quasisimultane Darstellung zu bekommen. Ein derartiges System ist allerdings sehr komplex und kostspielig. Außerdem kann es Probleme geben, wenn der Abtastkopf sich während der Zeit, in der Dopplereffekt - Wandler betrieben werden, bewegt, da solche Bewegungen zu Fehlzuordnungen zwischen der Dopperinformation und der gespeicherten B - Abtast - Information führen, wenn diese dargestellt wird.

Mit der Erfindung wird es dagegen möglich, nur einen einzigen Ultraschallwandler für beide Funktionen der Abbildung des Körperinneren und der Bestimmung der Blutströmungsgeschwindigkeit zu benutzen, womit gleichzeitig Ausrichtungsprobleme, wie sie bei der Verwendung zweier Wandler auftreten, von vornherein ausgeschlossen werden und die Kosten der Vorrichtung sich niedrig halten lassen.

Bei dem erfindungsgemäßen Verfehren wird ein fokussiertes Ultraschallstrahlenbündel mit einer gegebenen Apertur auf die Haut eines Körpers gerichtet, wobei der Zentralstrahl senkrecht auf der Haut steht. Das aus dem Körper reflektierte Ultraschallstrahlenbündel wird mit der gleichen Apertur empfangen. Zum gerichteten Ausstrahlen und Empfangen des Ultraschalls wird der gleiche und einzige Schallwandler verwendet, wobei das Strahlenbündel mit einer Linse fokussiert wird. In dem empfangenen Ultraschallstrahl werden Schwebungsfrequenzsignale festgestellt, die aus der Überlagerung von Anteilen resultieren, die durch den Dopplereffekt beeinflußt sind und von der bewegten Materie, beispielsweise Blutkörperchen des sich im Körper bewegenden Bluts reflektiert wurden. Als Funktion der Schwebungsfrequenzsignale wird danne eine geschwindigkeitsrepräsentative Anzeige, beispielsweise in hörbarer oder sichtbarer Form, erzeugt. Die Schwebungsfrequenzsignale entsprechen Anteilen des empfangenen Ultraschallstrahls, welche sowohl Frequenzkomponenten oberhalb als auch unterhalb der Frequenz des ursprünglich eingestrahlten Ultraschallstrahlenbündels haben.

Gemäß einer Weiterbildung der Erfindung läßt sich die Vorrichtung zur Messung der Flußgeschwindigkeit von Blut mit Hilfe des Dopplereffekts kombinieren mit einer Vorrichtung zur gleichzeitigen bildlichen Darstellung von Abschnitten des Körpers unter Benutzung desselben Schallwandlers. Eine solche kombinierte Vorrichtung enthält eine Zeitgeberschaltung zur Erzeugung von Zeittaktsignalen und eine Sende/Empfangseinrichtung, welche alternierend und durch die Zeittaktsignale gesteuert betrieben wird, und mit einem Schallwandler verbunden ist, der die elektrische Energie in ein Ultraschallstrahlenbündel umwandelt und umgekehrt. Das fokussierte Ultraschallstrahlenbündel wird senkrecht in die Haut des Körpers gestrahlt. Die reflektierten Ultraschallechos werden zurück auf den Schallwandler gelenkt. Die Ausgangssignale der Sende-/Empfangseinrichtung werden einer ersten Verarbeitungseinheit zur Erzeugung von Bildsignalen zugeleitet. Eine dem Ausgang der Sende-/Empfangseinrichtung verbundene zweite Verarbeitungseinheit ermittelt Schwebungsfrequenzsignale aus Komponenten, die von dem im Körper bewegten Blut reflektiert wurden. Die Schwebungsfrequenzsignale werden dann zu einer Geschwindigkeitsanzeige verarbeitet.

Schallwandler, Fokussiereinrichtung und Strahlführungselemente sind vorzugsweise in einem mit Flüssigkeit gefüllten tragbaren Meßkopf untergebracht. Die Strahlführungselemente können einen in der Flüssigkeit sich hin- und herbewegenden Schallspiegel enthalten oder einfach in einer entsprechenden Befestigung des Schallwandlers in seinem Gehäuse oder in dem tragbaren Meßkopf bestehen, die geometrisch so ausgelegt ist, daß der Zentralstrahl des fokussierten Ultraschallstrahlenbündels im wesentlichen senkrecht auf die Haut gerichtet ist.

Weitere Merkmale und Vorteile der Erfindung gehen aus der folgenden Beschreibung der Zeichnung hervor, die eine bevorzugte Ausführungsform der Erfindung darstellt.

Fig. 1 veranschaulicht die betriebsmäßige Anwendung einer erfindungsgemäßen Vorrichtung.

Fig. 2 ist eine perspektivische Ansicht des Abtastkopfes einer Vorrichtung nach Fig. 1.

Fig. 3 zeigt eine Querschnittsdarstellung eines Abtastkopfes nach Fig. 2 entlang einer durch die Pfeile 3—3 definierten Ebene und ein schematisiertes Blockschaltbild von Teilen der in dem Abtastkopf und in der zugehörigen Konsole untergebrachten elektronischen Schaltung.

Fig. 4 ist eine vereinfachte Prinzipdarstellung eines fokussierten Ultraschallstrahls, der auf ein subkutanes Blutgefäß gerichtet ist.

Fig. 5 zeigt ein zweidimensionales B - Abtastungsbild eines Querschnitts durch eine menschiche Karotisarterie.

Fig. 6 zeigt eine vertikal gespreizte eindimensionale B - Abtanstungsdarstellung, wie sie aus einer einzelnen Abtastzeile nach Fig. 5 erhalten wird.

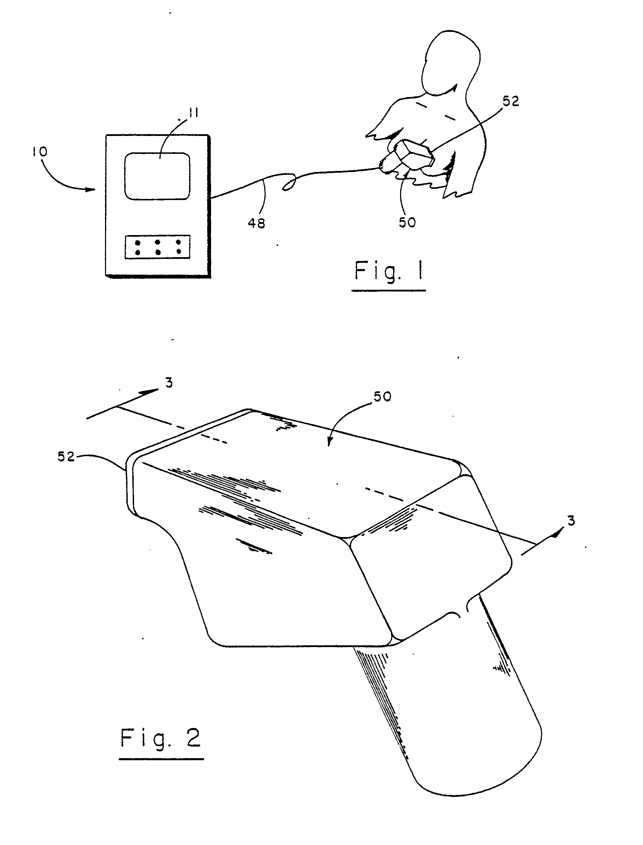

In Fig. 1 ist eine Anwendung einer Abtastvorrichtung gekäß der Erfindung dargestellt. Ein Anzeige- und Kontrollgerät 10 ist mit einem Sichtgerät 11 versehen, das üblicherweise eine Kathodenstrahlröhre ähnlich wie beim Fernsehen sein kann. Weiter weist das Anzeige- und Kontrollgerät 10 ein Bedienungsfeld auf. Auch eine Videoband·- Aufzeichnungseinrichtung oder eine geeignete photografische Vorrichtung kann zu dem Anzeige- und Kontrollgerät 10 gehören. Das Anzeige- und Kontrollgerät wird üblicherweise die Stromversorgung und Teile der Zeittakt- und Verarbeitungsschaltung des im folgenden beschriebenen Systems einschließen. Ein tragbarer Abtastkopf 50 (s. auch Fig. 2) ist über ein Kabel 48 an das Anzeigeund Kontrollgerät 10 angeschlossen. Der Abtastkopf hat ein Abtastfenster 52 bei einem seiner Enden, durch das ein Ultraschallstrahlenbündel zu Untersuchungszwecken ausgesandt und der reflektierte Ultraschall empfangen wird. Im Betrieb der Vorrichtung wird der Abtastkoft 50 von Hand in eine Position gebracht, bei der das Abtastfenster 52 über dem darzustellenden Körperteil angeordnet ist. In Fig. 1 ist beispielsweise der Abtastkopf in eine solche Position gebracht, daß ein Querschnittsbild der Brust erhalten wird. Bildliche Darstellungen anderer Körperteile kann man leicht erhalten, indem man den Abtastkopf 50 in die gewünschte Position und Orientierung bringt, wobei die relative Orientierung des Abtastfensters 52 die Winkellage des zu bestimmenden Querschnitts festlegt.

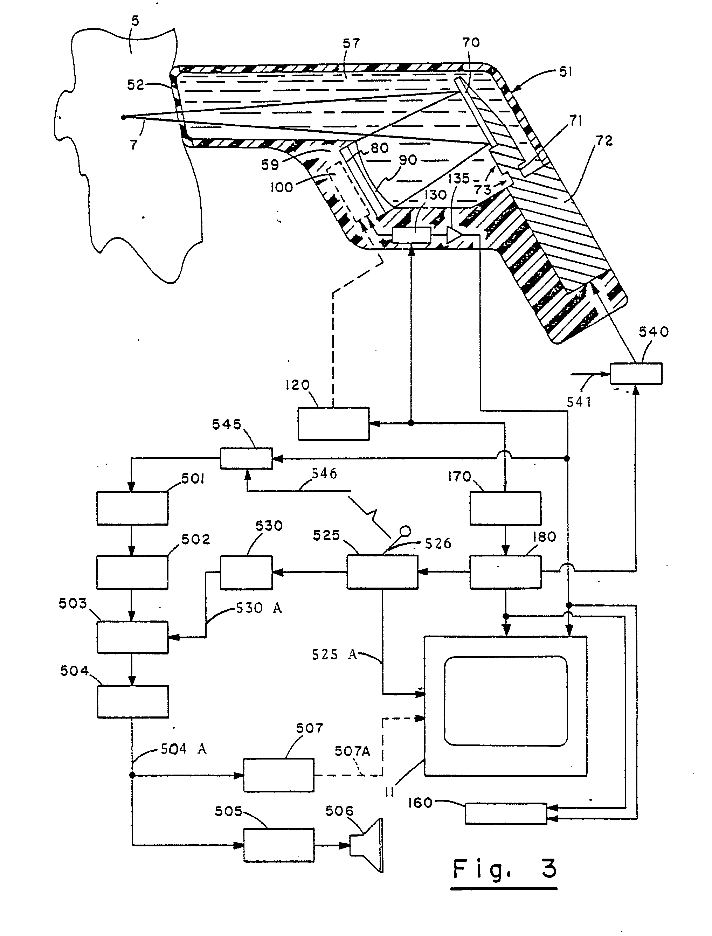

In Fig. 3 ist im wesentlichen eine Querschnittsdarstellung eines Teils des Abtastkopfes 50 zusammen mit Diagrammen von Teilen der in dem Abtastkopf und in dem Anzeige- und Kontrollgerät 10 enthaltenen Schaltung wiedergegeben. Ein flüssigkeitsdichtes Gehäuse 51, das aus einem stabilen Kunststoffmaterial hergestellt sein kann, weist ein·Abtastfenster 52 an seinem Vorderende auf. Der Abtastkopf 50 wird mit dem Abtastfenster gegen einen Körperteil 5 gehalten.

Das Gehäuse 51 ist mit einer geeigneten Flüssigkeit 57, beispielsweise Wasser gefüllt. Das Abtastfenster 52 ist im wesentlichen flach und kann beispielsweise aus Methylmethacrylat oder einem Polyamid bestehen. Eine schallreflektierende Abtasteinrichtung 70, die im folgenden als Abtastspiegel bezeichnet wird und in der Zeichnung flach dargestellt ist, aber auch gekrümmt sein kann, um zugleich eine fokussierende Wirkung zu haben, ist in der Nähe des rückwärtigen Endes des Gehäuses 51 angeordnet und in wesentlichen auf das Abtastfenster 52 gerichtet.

Der Abtastspiegel 70 ist an einer Achse 71 befestigt, die eine geeignete Dichtung durchdringt und mit einem elektrischen Motor 72 verbunden ist, der in einer Ausnehmung des Gehäuses 51 angebracht ist. Die Achse 71 wird so angetrieben, daß die gewünschte Hin- und Herbewegung (oscillatory motion) des Abtastspiegels 70, angedeutet durch den gekrümmten zweiköpfigen Pfeil 73, erreicht wird.

Ein Ultraschallwandler 80 ist in einem Abteil 59 des Gehäuses 51 angeordnet. Der Wandler ist bezüglich des Abtastspiegel 70 in Vorwärtsrichtung in dem Antastkopf 50 untergebracht, wobei die den Ultraschall abstrahlende Fläche des Wandlers im wesentlichen in dem Abtastkopf 50 rückwärts weist und auf den Abtastspiegel 70 gerichtet ist. Der Schallwandler 80 ist so positioniert, daß das von ihm ausgesandte Ultraschallstrahlenbündel 7 von dem Abtastspiegel 70 reflektiert wird und an dem Wandler 80 vorbei zurückfällt, bevor es durch das Abtastfenster 52 austritt. Die reflektierende Oberfläche des Abtastspiegels ist bevorzugt aus einem Material wie Saphir hergestellt, das in Verbindung mit dem umgebenden Wasser zu einem relativ kleinen kritischen Winkel führt, so daß auch ein nahezu senkrecht auf die Abtastspiegeloberfläche auftreffender Strahl reflektiert wird und nicht den Reflektor durchdringt.

Eine Impulssende-/Empfangsschaltung 130 liefert alternierend Energieimpulse an den Schallwandler 80 und empfängt Echosignale von diesem. Der Begriff Impulssende-/Empfangsschaltung soll hier so verstanden werden, daß er jede kombinierte oder getrennte Schaltungsanordnung einschließt, die dazu dient, Betriebssignale für den Schallwandler zu erzeugen und Echosignale von ihm zu empfangen. Wenn dynamische Fokussierung angewandt wird, kann der Schallwandler 80 in Abschnitte unterteilt sein und die Impulssende-/Empfangschaltung 130 kann mit den Abschnitten des Schallwandlers 80 über eine Schaltung zur variablen Verzögerung 100 verbunden sein, wie sie in Fig. 3 gestrichelt dargestellt ist. Die Impulssende-/Empfangsschaltung 130 und die Schaltung zur variablen Verzögerung 100 (falls vorhanden) sind typischerweise, aber nicht notwendig, in dem Abtastkopf 50 untergebracht, beispielsweise in dem Gehäuseteil 59. Der Empfangsteil der Schaltung 130 ist über einen Vorverstärker 135, der vorzugsweise in dem tragbaren Abtastkopf 50 angeordnet ist, mit einem Sichtgerät 11 und einem Aufnahmegerät 160 verbunden. Als Aufnahmegerät kann jede geeignete Aufnahme-, Speicher- und/oder photografische Einrichtung, beispielsweise ein Video - Aufnahmegerät verwendet werden. Die Zeitgeberschaltung 170 erzeugt Zeittaktsignale, die den Betrieb des Systems synchronisieren. Die Zeittaktsignale sind an den Impulssender-/Empfänger 130 und ebenso an die Ablenkschaltung 180 angelegt. Die Ablenkschaltung 180 erzeugt Signale, die die Hin- und Herbewegung des Abtastspiegels 70 kontrollieren, sowie die vertikalen und horizontalen Synchronisationssignale des Sichtgerätes 11 und der Aufnahmeeinrichtung 160. Bei Verwendung einer Einrichtung zur dynamischen Fokussierung, wie sie in der EP—A1—00 13 029 beschrieben sind, können die Zeittaktsignale zugleich an eine Phasenkontrollschaltung 120 angeschlossen sein, welche ihrerseits Signale zur Kontrolle der Veränderung der Verzögerungen in der Schaltung zur variablen Verzögerung 100 erzeugt.

In dem Abtastkopf 50 ist weiterhin eine Linse 90 angordnet, die typischerweise eine verhältnismäßig flache mit dem Schallwandler verbundene Oberfläche hat und deren andero Oberfläche typischerweise konkav gekrümmt ist, um eine axial-symmetrische Fokussierung zu erreichen. Die Linse kann beispeilsweise aus einem Kunststoffmaterial hergestellt sein, welches gemäß US—PS—3.958.559 ausgewählt ist. Wie in dieser Patentschrift beschrieben wird, erreicht man durch Auswahl des Linsenmaterials gemäß den spezifizierten Parametern, daß unerwünschte Seitenstrahlungskeulen, die durch Faktoren wie die endliche Größe des Schallwandlers erzeugt werden, werden auf ein Minimum reduziert. Weiterhin kann die Linse, wie in dem angegebenen Patent dargelegt, eine im wesentlichen elliptische Kontur haben, um eine vorteilhafte Charakteristik zu erreichen. Es können aber je nach den Einzelumständen auch andere Einrichtungen zur Fokussierung verwendet werden, beispielsweise eine elektronische Fokussierung mit Hilfe eines in Abschnitte unterteilten Schallwandlers. Auch eine entsprechende Krümmung des Schallwandlers oder der Oberfläche des Abtastspiegels kann zur Fokussierung verwendet werden.

Der bis jetzt beschriebene Teil des Systems arbeitet im Betrieb etwa folgendermaßen: Auf ein Kommandosignal der Zeitgeberschaltung erzeugt der Impulssender in der Schaltung 130 Impulse, die den Schallwandler 80 erregen. Dabei werden die Abschnitte des Schallwandlers 80 bei Verwendung von dynamischer Fokussierung über die Schaltung zur variablen Verzögerung 100 erregt, die ihrerseits Kontrollsignale von der Phasenkontrollschaltung 120 erhält. (Wie dem Fachmann wohlbekannt ist, kann in einem dynamisch fokussierten System die Tiefenlage des Fokuspunkts elektronisch variiert werden, indem man verschiedenen Abschnitten des Schallwandlers 80 die Signale mit verschiedenen vorbestimmten Verzögerungen oder Phasenverschiebungen zuführt. Üblicherweise ist in solch einem Fall die Schaltung zur variablen Verzögerung so eingestellt, daß beim Aussenden des Ultraschallimpulses das Strahlenbündel auf einen Punkt fokussiert ist, der zwischen der Mitte des möglichen Brennweitenbereiches und dem tiefsten Punkt in dem Körper liegt, für den ein Bild erzeugt werden soll). Der aus den dem Schallwandler zugeführten Impulsen resultierende Ultraschallstrahl wird von dem Reflektor 70 durch das Abtastfenster 52 in den Körper 5 reflektiert. Danach wird auf ein Signal der Zeitgeberschaltung 170 hin die Impulssende-/Empfangsschaltung 130 auf "Empfang" geschaltet. (Bei Verwendung dynamischer Fokussierung wird ein Zyklus der Phasenkontrollschaltung 120 eingeleitet). Die Ultraschallechos, die aus dem Körper 5 durch das Fenster 52 und zurückreflektiert von dem Abtastspiegel 70 auf den Schallwandler 80 fallen, werden jetzt von dem Schallwandler 80 in elektrische Signale umgewandelt. (Im Falle der Verwendung dynamischer Fokussierung wandeln die Einzelnen Abschnitte des Schallwandlers die empfangene Ultraschallenergie in elektrische Signale um, welche dann in geeigneter Phasenbeziehung zu einander zusammengefaßt werden, dergestalt, daß eine Fokussierung auf einen bestimmten Ursprungspunkt des reflektierten Ultraschalls im zu untersuchenden Tiefenbereich erreicht wird). Im Falle einer zweidimensionalen "B-Abtastung"-Darstellung entspricht ein Durchlauf Durch den Tiefenbereich einer horizontalen Abtastzeile auf dem Sichtgerät. Die horizontale Synchronisation des Sichtgerätes 11 muß daher durch die Zeitgeberschaltung 170 derart synchronisiert sein, daß der wirksame Teil einer Abtastzeile des Sichtgerätes 11 der Eintreffzeit von Echos aus einem vorgegebenen Tiefenbereich in dem Körper 5 entspricht, üblicherweise zwischen der Haut des Patienten und einer festen vorgewählten Tiefe in dem Körper 5. Die Zweite Dimension des gewünschten Querschnittsbildes erreicht man durch die langsamere mechanische Abtastbewegung des Abtastschaltspiegels 70, die ihrerseits durch die Ablenkschaltung 180 mit der Vertikalablenkung des Sichtgerätes 11 und des Aufnahmegerätes 160 synchronisiert ist. Die empfangenen Signale werden über den Verstäker 135 an das Sichtgerät 11 geleitet. Sie modulieren dort die Helligkeit des Zeilenrasters (scanning raster), um das gewünschte Querschnittsbild zu erhalten, wobei jede Abtastzeile des Sichtgerätes 11 dem Profil eines Tiefenechos in dem Körper für eine bestimmte Winkellage des Abtastspiegels 70 entspricht. Die empfangenen Signale werden auch von dem Video - Bandaufnahmegerät 160 aufgenommen.

Als nächstes wird das Untersystem zur Bestimmung der Geschwindigkeit bewegter Materie, üblicherweise Blut, in dem Körper beschrieben. Das Ausgangssignal des Vorverstärkers 135 liegt auch an dem Gatter 545 an, dessen Steuersignal (Leitung 546) manuell steuerbar ist. Das Ausgangssignal des Gatters 545 ist an einen Bandpaßfilter 501 angelegt. Der Filter hat eine Durchlaßbreite von etwa 1 MHz mit einer Mittenfrequenz von ungefähr 8 MHz, der Betriebsfrequenz des Schallwandlers 80 bei der dargestellten bevorzugten Ausführungsform. Das Ausgangssignal des Bandpaßfilters 501 ist an einen Radiofrequenz- (HF) Detektor 502 angelegt, dessen Ausgangssignal seinerseits an ein Gatter 503 anliegt, das als Teifenbereichsgatter (range gate) dient. Der Einschaltkontakt (enable terminal) des Gatters 503 (Leitung 530A) wird vom Ausgang eines Cursorgenerators über einen monostabilen Multivibrator 530 gesteuert. Der Ausgang des Tiefenbereichsgatters 506 ist mit einem Audiodetektor (audio detector) 504 verbunden, dessen Ausgangssignale wiederum an einem Niederfrequenzverstärker 505 und an einem Frequenzanalysator 507 anliegt. Der Niederfrequenzverstärker 505 ist mit einem Lautsprecher 506 verbunden. Der Frequenzanalysator 507 kann eine eigene in der Zeichnung nicht dargestellte Anzeigeeinheit aufweisen, oder er kann benutzt werden, um ein Videosignal zu erzeugen, welches über die gestrichelt dargestellte Leitung 507A an das Sichtgerät 11 angelegt wird, wo es eine Videoanzeige entsprechend dem Doppler-Ausgangsignal (Leitung 504A) erzeugt. Cursorgeneratoren sind dem Fachmann wohlbekannt und werden hier nicht im einzelnen beschrieben. Kurz gesagt erhält der Cursorgenerator 525 Synchronisationssignale von der Ablenkungsschaltung 180 und weist eine Einrichtung zur manuellen Kontrolle, beispielsweise einen Einstellknopf oder einen "Steuerknüppel" 526 auf, mit dessen Hilfe ein Referenzpunkt auf der der Anzeige des Sichtgerätes gewählt werden kann. Ein Videosignal zur Darstellung dieses Puntes wird an das Vldeo-Sichtgerät angelegt und erzeugt dort einen Bildpunkt, d.h. den Cursorpunkt. Das Cursor-Videosignal ist außerdem an den Einschaltkontakt des Gatter 503 über den monostabilen Multivibrator 530 und die Leitung 530A angelegt. Schließlich ist noch ein Gatter 540 vorgesehen, mit dessen Hilfe die Ablenkungssignale, die den Motor 72 kontrollieren, abgeschaltet werden können. Das Gatter 540 ist über die Lietung 541, bevorzugt zusammen mit dem Gatter 545 manuell steuerbar.

Im Betrieb arbeitet das Dopplereffekt-Untersystem kurz gesagt folgendermaßen: Das Betriebspersonal wählt mit Hilfe des Steuerknüppels 526 des Cursorgenerators 525 eine Position des Cursorpunktes auf dem Sichtgerät 11. Ebenfalls unter Kontrolle des Betriebspersonals wird über Leitung 546 dem Gatter 545 ein Signal zugeführt, so daß dieses öffnet und das Ausgangssignal des Vorverstärkers 135 an die Verarbeitungseinheit gelangen kann, die mit dem Filter 501 beginnt. Bei der dargestellten bevorzugten Ausführungsform wird zugleich unter Kontrolle des Betriebspersonals über Leitung 541 dem Gatter 540 ein Signal dergestalt zugeführt, daß die Abtastbewegung des Abtastspiegels 70 unterbrochen wird, und zwar so, daß der Spiegel in einer vorbestimmten Ruheposition stehenbleibt, typischerweise in der Mitte seines Abtastsektors. Die Dopplereffekt- Information wird von der Schaltung 501—504 empfangen und verarbeitet und über den Lautsprecher 506 und/oder dem Frequenzanalysator 507 wiedergegeben, wie im folgenden beschrieben wird. Gleichzeitig wird weiterhin eine B - Abtastungs - Information in einer Dimension (d.h. nur in die Tiefe, da der Abtastspiegel 70 gestoppt ist) auf dem Sichtgerät 11 dargestellt, wobei das Konventionell ausgeführte B - Abtastungs - Untersystem benutzt wird.

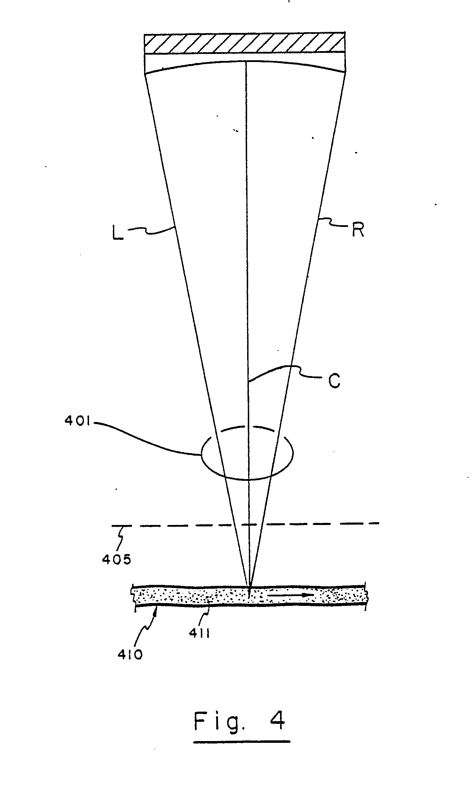

Vor der Beschreibung weiterer Einzelheiten der Betriebsweise des Untersystems zur Detektion des Dopplereffekt - beeinflußten Signals, werden im folgenden anhand der vereinfachten schematischen Darstellung nach Fig. 4 einige Ausführungen gemacht, die in vereinfachender Art und Weise ein Verständnis des erfindugsgemäß detektierten, d.h. erfaßten und mit elektronischen Mitteln ausgewerteten, Dopplereffekts ermöglichen sollen. Ein fokussiertes Ultraschallstrahlenbündel 401 ist auf das sich in einem Blutgefäß 410 bewegende Blut 411 gerichtet, wobei das Blutgefäß im wesentlichen parallel zur Haut 405 des Patienten verläuft. Der mit dem Bezugzeichen C bezeichnete Zentralstrahl des Strahlenbündels ist im wesentlichen senkrecht auf die Haut 405 gerichtet, so daß die entlang diesem Zentralstrahl zurückgestreute Ultraschallenergie keiner nennenswerten Doppler - Frequenzverschiebung unterliegt. (Aus diesem Grunde wird bei den Geschwindigkeits - Meßsystemem gemäß dem Stand der Technik im allgemeinen ein Strahlenbündel in einem Winkel bezüglich der Senkrechten zur Bewegungsrichtung der Materie, deren Geschwindigkeit gemessen werden soll, ausgerichtet). Betrachtet man nun die Ultraschallenergie an den äußeren Begrenzungen des fokussierten Strahlenbündels, beispielsweise die mit den Bezugzeichen L und R in der Fig. 4 versehenen Außenstrahlen des fokussierten Strahlenbündels, so hat der Strahl L eine Komponente in der Richtung des Blutflusses, während der Strahl R eine Komponente entgegen der Richtung des Blutflusses hat. Demzufolge wird Ultraschallenergie, die entlang dem Strahl L auf den Shallwandler zurückgestreut wird aufgrund von Doppler - Verschiebung eine niedrigere Frequenz haben, als die ursprünglich ausgestrahlte Ultrachallenergie (die man als "Träger" bezeichnen kann). Im Gegensatz dazu wird Ultraschallenergie, die entlang dem Strahl R auf den Schallwandler 80 zurückgestreut wird, aufground des Dopplereffekts eine höhere Frequenz als der Träger hanen.

Die Frequenzen oberhalb und unterhalb der Trägerfrequenz überlagern einander und die aus der Überlagerung resultierende Schwebungsfrequenz (the beats) kann detektiert werden. Die Schwebungsfrequenz ist eine Funktion der Geschwindigkeit der zurüskstreuenden oder reflektierenden Materie und kann daher als Maßstab für die Geschwindigkeit binutzt werden. Für den Fall einer rechteckigen Apertur und für die vereinfachte Situation nach Fig. 4, kann die Doppler - Frequenzverschiebung $\Delta F$ oberhalb oder unterhalb der Trägerfrequenz näherungsweise berechnet werden zu:

$$\Delta F = \frac{F_0 V}{2f \cdot C}$$

Darin bezeichnet $F_0$ die Trägerfrequenz, f die relative Öffnung des Brennkegels, (f-number of the focused cone), C die Geschwindigkeit des Ultraschalls im Blut und V die zu messende Blutgeschwindigkeit. Für einen runden Schallwandler ist im Nenner der Faktor 2 durch einen Faktor 2,4 zu ersetzen. Wie man sieht, ist die Doppler - Frequenzverschiebung umgekehrt proportional der relaiven Öffnung f des Strahlkegels. Für eine bestimmte Brennweite ist daher die Apertur des fokussierten Ultraschallstrahlenbündels die bestimmende Größe für den Frequenzbereich der Doppler - Verschiebung. Je größer die Apertur (d.h. je kleiner die Zahl f), desto höher ist die Betriebsfrequenz. Der Schallwandler kann eine beliebige andere Form haben, beispielsweise kann er gemäß der DE—A1—29 11 613 eliptisch ausgeführt sein. Dadurch wird die obige Beziehung zur Bestimmung der Doppler - Frequenzverschiebung modifiziert, jedoch kann die jeweils gültige Beziehung zwischen Frequenzverschiebung und Blutgeschwindigkeit empirisch selbst für ungewöhnliche Konfigurationen des Ultraschallwandlers 80 bestimmt werden. Obwohl Fig. 4 in einem vereinfachten Beispiel ein nicht reflektiertes Ultraschallstrahlenbündel zeigt, gelten die gleichen prinzipiellen Überlegungen selbstverständlich für eine Ausführungsform gemäß Fig. 3, bei der das Ultraschallstrahlenbündel von der Oberfläche des Abtastspiegels 70 reflektiert wird.

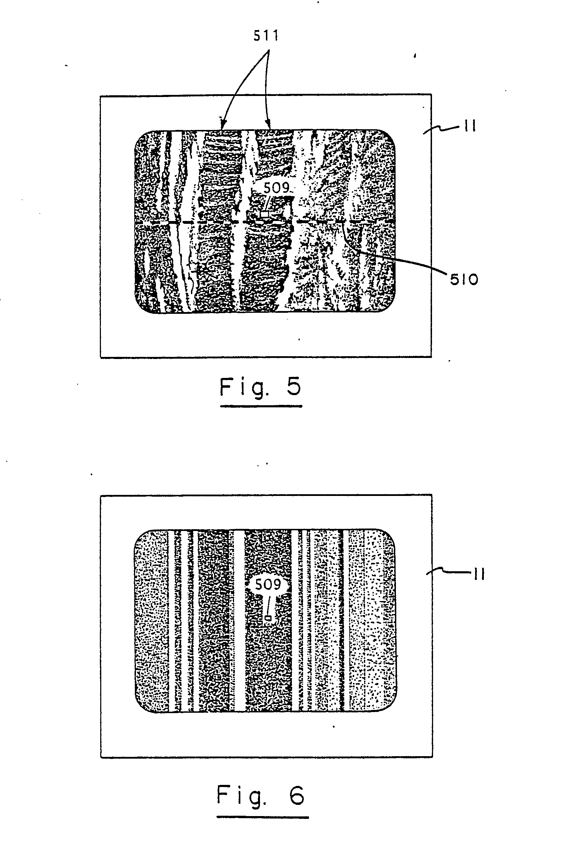

Im folgenden werden weitere Einzelheiten der Betriebsweise eines Systems gemäß Fig. 3 beschrieben. Wenn simultan mit einer B - Abtastungs - Darstellung eine Geschwindigkeitsinformation gewünscht wird, kann das Betriebspersonal den Steuerknüppel 526 des Cursorgenerators 525 so einstellen, daß der Cursorpunkt auf der B - Abtastungs - Darstellung des Sichtgerätes 11 eine einem bestimmten Punkt im Körper entsprechende Position einnimmt. In der Darstellung nach Fig. 5 ist beispielsweise ein Cursorpunkt 509 in der Karotisarterie 511 dargestellt, die man in einem zweidimensionalen B - Abtastungsbild eines Querschnitts durch einen menschlichen Hals erkennt. Wie bereits weiter oben beschrieben, wird durch einen vom Betriebspersonal ausgelösten Befehl das Gatter 545 geöffnet und das Gatter 654 geschlossen, was beispielsweise über einen nichtdargestellten gemeinsamen Schalter erreicht werden kann. Da somit der Abtastspiegel 70 angehalten wird, wird die B - Abtastungs - Darstellung auf dem Sichtgerät 11 naturgemäß eindimensional. Das heißt, daß in horizontaler Richtung in Fig. 6 die Helligkeit der Anzeige als Funktion ihrer Position der relativen Stärke der aus der entsprechenden Tiefe des Körpers reflektierten bzw. gestreuten Echos entspricht. Da die Anzeige des Sichtgerätes 11 selbst wegen der Vertikalablenkung des Gerätes weiterhin zweidimensional ist, wird die eindimensionale Helligkeitinformation in vertikaler Richtung gespreizt, wobei auf der Anzeige des Sichtgerätes unabhängig von der vertikalen Position in vertikaler Richtung überall die gleiche Information dargestellt wird. Man betrachte hierzu die ungefähr in der Mitte liegende vertikale Zeile des Sichtgerätes 11, die in Fig. 5 durch die gestrichelte linie 510 markiert ist. Diese Abtastzeile des Sichtgerätes entspricht der B - Abtastungsinformation in der Mittelstellung des Abtastspiegels 70, die bei geschlossenem Gatter 540 die Ruheposition des Abtastspiegels 70 ist. Das bei geschlossenem Gatter 540 und somit stehenden Abtastspiegel 70 sich ergebende vertikal - gespreizte eindimensionale B - Abtastungsbild ist in Fig. 6 dargestellt. Fig. 6 ist also vereinfacht ausgedrückt eine vertikal gespreizte Darstellung der Abtastzeile 510 aus Fig. 5. Der Cursorpunkt, der das Tiefenbereichgatter des Doppler - Effekt - Untersystems kontrolliert, kann jederzeit vor und/oder nach dem Schließen des Gatters 540 in gewünschter Weise eingestellt werden.

Nachdem das Gatter 545 geöffnet und somit die Detektion des Doppler-Effektes mit Hilfe des entsprechenden Untersystems möglich ist, werden dem Bandpaßfilter 501 die breitbandig verstärkten Ausganssignale der Impuls sende-/Empfangsschaltung 130 zugeführt. Der Bandpaßfilter 501 filtert dieses Signal auf eine Bandbreite von ungefähr 1 MHz mit einer Mittenfrequenz von ungefähr 8 MHz, der charakteristischen Betriebsfrequenz des Schallwandlers 80 in der dargestellten bevorzugten Ausführungsform. Der RF-Detektor 502 detektiert die Einhüllende des ausgefilterten Signals und das Tiefenbereichsgatter 503 selektiert einen entsprechenden zeitlichen Abschnitt aus jedem Echo, entsprechend der vom Betriebspersonal mit Hilfe des Steuerknüppels vorgewählten Position des Cursorpunktes. Die Länge dieses Zeitabschnittes ist bestimmt durch die Einschaltdauer des monostabilen Mulivibrators 530 und liegt typischerweise bei etwa 1,3 Mikrosekunden (entsprechend einer Tiefenerstreckung von etwa 1 mm in dem Körper). Der zeitliche Abschnitt kann aber wahlweise entsprechend jeder gewünschten Tiefenerstreckung eingestellt werden. Das entsprechend einem bestimmten Eindringtiefenabschnitt ausgeschnittene Ausgangssignal des Gatters 503 wird an einen Audio-Detektor 504 angelegt, dessen Ausganssignal wiederum an einen Niederfrequenzverstärker 505 und danach an einen Lautsprecher 506 geleitet wird, sowie gleichzeitig an den Frequenzanalysator 507. Das Ausgangssignal des Frequenzanalysators 507 kann an das Sichtgerät 11 angelegt werden, um es gemeinsam mit der B - Abtastungsinformation darzustellen. Dies ist durch die gestrichelt dargestellte Verbindungsleitung 507A angedeutet.

Die Erfindung wurde vorstehend unter Bezug auf eine besondere Ausführungsform beschrieben, dem Fachmann sind jedoch zahlreiche weitere Ausgestaltungen des Erfindungsgedankens unmittelbar zugänglich. Während beispielsweise bei der Ausführungsform nach Fig. 3 das Dopplereffekt - Ausganssignal bei vorübergehend gestoppten Abtastspiegel gewonnen wird, könnte ein Dopplereffekt - Ausgangssignal auch gewonnen werden, während gleichzeitig die zweidimensionale B-Abtastung fortgesetzt wird, wobei dann eine geeignete Filtereinrichtung benutzt werden müsste. um die Frequenz der Abtastbewegung zu entfernen. Vorzugsweise könnte ein Kammfilter verwendet werden, um die Frequenz der Abtastbewegung auszufiltern. Das Dopplereffekt - Ausgangssignal das mit einem solchen System gewonnen würde, wäre wegen der Geschwindigkeit der Bewegung des Abtaststrahls selbst nicht das Gleiche wie mit einem stationären Strahlenbündel. Eine Kammfilterung zur Entfernung der immer wiederkehrenden Signalform kann man z.B erreichen, indem man das niederfrequente Ausgangssignal um die charakteristische Frequenz der Abtastbewegung verzögert, invertiert und dann zu dem nicht - verzögerten Signal addiert.

## Patentansprüche

1. Betriebsverfahren für einer Vorrichtung zur Bestimmung der Geschwindigkeit von bewegter Materie im Körper, mit einem einen Ultraschallwendler aufweisenden Meßkopf, der ein fokussiertes Ultrachallstrahlenbündel mit einer gegebenen Apertur auf die Hautoberfläche des Körpers ausstrahlt und das reflektierte Ultraschallstrahlenbündel über die gleiche Apertur aus dem Körper wieder empfängt und mit einer Signalverarbeitungsschaltung, welche Schwebungsfrequenzsignale, die aus von der bewegten Materie reflektierten Dopplereffektkomponenten resultieren, ermittelt und zu einer Geschwindigkeitsanzeige verarbeitet, dadurch gekennzeichnet, daß der Zentralstrahl (C) des Strahlenbündels (401) im wesentlichen senkrecht zu der Haut oberfläche (405) des Körpers gerichtet wird und die reflektierten Ultraschallstrahlen mit dem gleichen, zum Aussenden benutzen Ultraschallwandler empfangen werden und daß die Schwebungsfrequenzsignale, die sich aus der Überlagerung der oberhalt und oder unterhalb der Aussendefrequenz des Ultraschallstrahlenbündels liegenden Doppler effektkomponenten ergeben zu der Geschwindigkeitsanzeige verarbeitet werden.

2. Betriebsverfahren nach Anspruch 1, dadurch gekennzeichnet, daß die relative Öffnung des fokussierten Ultraschallstrahlenbündels mindestens f/8 beträgt.

3. Betriebsverfahren nach Anspruch 1 oder 2, dadurch gekennzeichnet daß bei der Feststellung der Schwebungsfrequenzsignale nur ein bestimmter Eindringtiefenabschnitt ausgewählt wird.

4. Vorrichtung zur Durchführung des Betriebsverfahrens nach Anspruch 1, mit
—einem Ultraschallwandler,
—einer Sende-/Empfangseinrichtung, die abwechselnd den Ultraschallwandler mit Energie versorgt und die vom Ultraschallwandler kommende Energie empfängt,

—einer Fokussiereinrichtung für die vom Wandler abgestrahlte Ultraschallenergie,
—einer Strahlführungseinrichtung, die das fokussierte Ultraschallstrahlenbündel zur Haut des Körpers führt,
—einem Detektor zur Ermittlung der Schwebungsfrequenzsignale im Ausgangssignal des Ultraschallwandlers,
—einer Auswertschaltung für das Ausgangssignal des Detektors,
—und einer Anzeigeeinrichtung zur Erzeugung einer Anzeige als Maß für die Geschwindigkeit des in dem subkutanen Blutgefäß sich bewegenden Blutes,
gekennzeichnet durch die Kombination mit einem B-Abtaster für eine Querschnittsdarstellung von Teilen des Körpers durch Einstrahlung von Ultraschallenergie in den Körper und Bestimmung der charakteristischen Eigenschaften der aus dem Körper reflektierten Ultraschallenergie mit
—einem einzigen Ultraschallwandler (80),
—einer Zeitgeberschaltung (170) zur Erzeugung von Zeittaktsignalen für die Steuerung der Sende-/Empfangseinrichtung (130),
—einer ersten Auswertschaltung (135, 180) für das Ausgangssignal der Sende-/Empfangseinrichtung (130) zur Erzeugung eines Bildsignals, welches dem für den Körper charakteristischen Ultraschallecho entspricht,
—und einer zweiten Auswertschaltung (501—504) für das Ausgangssignal der Sende-/Empfangseinrichtung zur Ermittlung von Schwebungsfrequenzsignalen, welche aus der Überlagerung von dopplereffektbeeinflußten Komponenten resultieren, die von der bewegten Materie im Körper reflektiert sind.

5. Vorrichtung nach Anspruch 4, dadurch gekennzeichnet, daß der Detektor bzw. die zweite Auswertschaltung (501—504) für die Erfassung von Frequenzkomponenten oberhalb und unterhalb der Frequenz der vom Ultraschallwandler (80) ausgesandten Ultraschallenergie ausgebildet ist.

6. Vorrichtung nach Anspruch 4 oder 5, dadurch gekennzeichnet, daß der Schallwandler (80) eine zur Fokussierreinrichtung gehörende Linse (90) und die Strahlführungseinrichtung (70) in einem flüssigkeitsgefüllten tragbaren Meßkopf (50) angeordnet ist.

7. Vorrichtung nach Anspruch 6, dadurch gekennzeichnet, daß die Strahlführungseinrichtung einen in der Flüssigkeit angeordneten Abtastspiegel (70) enthält.

8) Vorrichtung nach Anspruch 7, gekennzeichnet durch eine von den Zeittaktsignalen synchronisierte Steuereinrichtung (180) für die Abtastbewegung des Abtastspiegels (70) und einen handbetätigbaren Schalter (540) zur Unterbrechung der Bewegung des Abtastspiegels, wenn die zweite Auswertschaltung (501—504) im Betrieb ist.

9. Vorrichtung nach einem der Ansprüche 4 bis 8, gekennzeichnet durch

—einen handsteuerbaren Cursorgenerator (525),

—eine mit dem Cursorgenerator synchronisierte Cursordarstellungseinrichtung zur Anzeige der gewählten Cursorposition auf einem Sichtgerät (11) für die Darstellung der Körperteile, und

—eine in der zweiten Auswertschaltung (501—504) enthaltene, mit dem Cursorgenerator synchronisierte Ausschneideschaltung (503, 530) für einen einem bestimmten Eindringtiefenabschnitt entsprechenden Signalteil.

## Revendications

1. Procédé d'exploitation pour un dispositif de détermination de la vitesse d'une matière en mouvement dans le corps, au moyen d'une tête de mesure présentant un transducteur ultrasonore qui dirige un faisceau focalisé de rayons ultrasonores, ayant une ouverture donnée, sur la face externe de la peau du corps et qui recueille le faisceau de rayons ultrasonores réfléchi en retour par le corps à travers la même ouverture, et comprenant également, et un circuit de traitement de signaux qui détermine des signaux de fréquence de battement qui résultent des composantes d'éffet Doppler réfléchies à partir de la matière en mouvement, et qui les transforme en une indication de vitesse, caractérisé en ce que le rayon central (C) du faisceau de rayons (401) est dirigé pratiquement perpendiculairement à la surface externe de la peau (405) du corps et les rayons ultrasonores réfléchies sont reçus par le méme transducteur ultrasonore utilisé pour l'émission, et en ce que les signaux de fréquence de battement qui résultent de la superposition des composantes d'effet Doppler se trouvant au-dessus et/ou en dessous de la fréquence d'émission du faisceau de rayons ultrasonores, sont transformés en une indication de vitesse.

2. Procédé suivant la revendication 2 caractérisé en ce que l'ouverture relative du faisceau de rayons ultrasonores focalisé est d'au moins f/8.

3. Procédé suivant l'une quelconque des revendications 1 et 2 caractérisé en ce que pour fixer les signaux de fréquence de battement on choisit uniquement une section de profondeur de pénétration déterminée.

4. Dispositif pour la mise en oeuvre de procédé suivant la revendication 1, comportant un transducteur ultrasonore, un dispositif d'émission/réception qui alternativement alimente le transducteur ultrasonore en énergie et reçoit l'énergie provenant du transducteur ultrasonore, un dispositif de focalisation pour l'énergie ultrasonore rayonnée à partir du transducteur ultrasonore, un dispositif de guidage du rayonnement qui conduit le faisceau de rayons ultrasonores focalisé vers la peau du corps, un détecteur pour déterminer les signaux de fréquence de battement dans le signal de sortie du transducteur ultrasonore, un circuit convertisseur pour le signal de sortie du détecteur, et un dispositif indicateur pour produire une indication correspondant à la mesure de la vitesse du sang se déplaçant dans le vaisseau sanguin subcutané, caractérisé par la combinaison, avec un dispositif de sondage du type B pour une représentation en coupe transversale de parties du corps par pénétration par rayonnement d'énergie ultrasonore dans le corps et détermination des propriétés caractéristiques de l'énergie ultrasonore réfléchie à partir du corps, d'un transducteur ultrasonore unique (80), d'un générateur de base de temps (170) pour la production de signaux de temporisation destinés à la commande du dispositif d'émission/réception (130), d'un premier circuit convertisseur (135, 180) pour le signal de sortie du dispositif d'émission/réception (130), lequel produit un signal d'image qui correspond à l'écho ultrasonore caractéristique pour le corps, et d'un second circuit convertisseur (501, 504) pour le signal de sortie du dispositif d'émission/réception afin de déterminer des signaux de fréquence de battement qui resultent de la superposition de composantes influencées par l'effet Doppler qui sont réfléchies à partir de la matiére en mouvement dans le corps.

5. Dispositif suivant la revendication 4 caractérisé en ce que le détecteur ou le second circuit convertisseur (501, 504) est prévu pour la saisic de composantes de fréquence se trouvant au-dessus et au–dessous de la fréquence de l'énergie ultrasonore émise à partir du transducteur ultrasonore (80).

6. Dispositif suivant l'une quelconque des revendications 4 ou 5 caractérisé en ce que le transducteur ultrasonore (80) est accolé à une lentille (90) appartenant au dispositif de focalisation et le dispositif de quidage du rayonnement (70) est disposé dans une tête de mesure portable (50) remplie de liquide.

7. Dispositif suivant la revendication 6 caractérisé en ce que le dispositif de guidage du rayonnement comporte un miroir de balayage (70) disposé dans le liquide.

8. Dispositif suivant la revendication 7 caractérisé en ce qu'il comporte un dispositif de commande (180) synchronisé par les signaux de temporisation, pour commander le mouvement de balayage du miroir de balayage (70), et un interrupteur (50) à commande manuelle pour interrompre le mouvement du miroir de balayage lorsque le second circuit convertisseur (501, 504) est en fonctionnement.

9. Dispositif suivant l'une quelconque des revendications 4 à 8 caractérisé en ce qu'il comprend un générateur de curseur (525) à commande manuelle, un dispositif de représentation d'un curseur synchronisé avec le générateur de curseur, afin d'afficher la position choisie du curseur sur un écran pour la représentation des parties du corps, et un circuit de coupure (503, 530) contenu dans le second circuit convertisseur (501, 504) et synchronisé avec le générateur de curseur, pour une partie de signal correspondant à une section donnée de profondeur de pénétration.

**Claims**

1. A method for operating an apparatus for determining the velocity of material moving in a body, comprising a scanning module including an ultrasound transducer means transmitting a focused ultrasound beam with a given aperture to the skin of said body and receiving the reflected ultrasound beam from said body over the same aperture, and signal processing means detecting beat frequency signals resulting from Doppler effect components reflected by said moved material, and processing such signals to provide a velocity representative indication, characterized in that the central ray (C) of said ultrasound beam (401) is being directed substantially normal to said skin surface (405) and said reflected ultrasound beam is being received by the same ultrasound transducer means (80) as used for transmission, and in that said beat frequency signals resulting from superimposing said Doppler effect signal portions above and/or below the transmission frequency of said ultrasound beam, are being processed to provide said velocity representative indication.

2. The method of claim 1, characterized in that the relative aperture of said focussed ultrasound beam is at least f/8.

3. The method of claim 1 or 2, characterized by selecting only a predetermined range of penetration for determining beat frequency signals.

4. Apparatus for conducting the method of claim 1, comprising
—an ultrasound transducer,
—transmitter/receiver means for alternately providing said ultrasound transducer with energy and receiving energy coming from said transducer,
—focusing means for focusing the ultrasound energy radiated by said transducer,
—beam guide means for guiding said focused ultrasound beam to the skin of said body,
—detector means for detecting beat frequency signals in the output signal of said ultrasound transducer,
—a utilizing circuit for processing the output signal to said detector means,
—and indicator means for generating an indication representative of the velocity of the blood moving in said subcutaneous blood vessel,
characterized by the combination with a B-scanner for cross-section imaging of portions of said body by radiating ultra-sound energy into said body and determining the characteristics of ultrasound energy reflected from said body, comprising
—a single ultrasound transducer (80),
—timing means (170) generating timing signals for controlling said transmitter/receiver means (130),
—first signal processing means (135, 138) for processing the output signal of said transmitter/receiver means (130) for generating an imaging signal representative of the ultrasound echo characteristics of said body,
—and second signal processing means (501—504) for processing the output signal of said transmitter/receiver means for detecting beat frequency signals resulting from beats of Doppler effect components reflected from moving material within said body.

5. Apparatus according to claim 4, characterized in that said detector means and said second signal processing circuit (501—504), respectively, are operative for processing frequency components above and below the ultrasound frequency as transmitted from said ultrasound transducer (80).

6. Apparatus according to claim 4 or 5, characterized in that said ultrasound transducer (80), a focusing lens (90) of said focusing means, and said beam guide means (70) are contained in a liquid-filled portable modul (50).

7. Apparatus according to claim 6, characterized in that said beam guide means comprises a scanner mirror (70) contained in said liquid.

8. Apparatus according to claim 7, characterized by control means (180) being synchronized with said timing signals for controlling the scan movement of said scanner mirror (70) and by operator controllable switch means (540) for interrupting the movement of said scanner mirror (70) when said second signal processing circuit (501—504) is operating.

9. Apparatus according to one of claims 4—8, characterized by
—an operator controllable cursor generator (525),
—cursor indicator means synchronized with said cursor generator for indicating the selected position of said cursor on a display (11) for imaging portions of said body,
—and said second signal processing circuit (501—504) including range gating means (503, 530) being synchronized with said cursor generator for selecting a signal portion corresponding to said predetermined range.

Fig. 1

Fig. 2

Fig. 3

Fig. 4

Fig. 5

Fig. 6